# EUROPEAN PATENT SPECIFICATION

(11) **EP 1 876 963 B1**
(45) Date of publication and mention of the grant of the patent: **02.01.2019**
(21) Application number: 06751253.3
(22) Date of filing: 25.04.2006
(51) Int. Cl.: A61B 17/00, A61B 17/34, A61B 18/00

(54) **DEVICES FOR PERICARDIAL ACCESS**
VORRICHTUNGEN FÜR DEN PERIKARDIALEN ZUGANG
DISPOSITIFS D'ACCES AU PERICARDE

(30) Priority: 27.04.2005 US 115408
(43) Date of publication of application: 16.01.2008
(73) Proprietor: Edwards Lifesciences AG, 1260 Nyon (CH)
(72) Inventor: VIDLUND, Robert, M., Forest Lake, Minnesota 55025 (US); SANTER, Jeffrey, D., Spring Lake Park, Minnesota 55432 (US); EKVALL, Craig, A., East Bethel, MN 55092 (US); SCHWEICH, Jr., Cyril, J., Maple Grove, Minnesota 55311 (US)
(74) Representative: Eisenführ Speiser
(86) International application number: PCT/US2006/015470
(87) International publication number: WO 2006/116310

(56) References cited:
- WO-A-03/066147
- WO-A1-03/066147
- WO-A1-03/066147
- US-A1- 2002 026 094
- US-A1- 2003 093 104
- US-A1- 2003 093 104
- US-A1- 2003 093 104
- US-B1- 6 231 518
- US-B1- 6 231 518
- US-B1- 6 231 518
- US-B1- 6 237 605
- US-B1- 6 237 605

## Description

### FIELD OF THE INVENTION

The present disclosure relates to devices and associated methods for less invasively accessing the heart. More particularly, the disclosure relates to devices and methods for accessing the pericardial space around the heart.

### BACKGROUND OF THE INVENTION

Access to the outside (epicardial) surface of the heart for various therapeutic and diagnostic purposes is typically achieved using a surgical technique. For example, placement of epicardial leads for electrophysiological applications has been historically performed surgically. However, surgically accessing the heart necessarily involves a certain amount of invasiveness and associated trauma, both of which are desirably minimized.

A variety of less invasive techniques for accessing the epicardial surface of the heart have been proposed in the prior art. These techniques focus, at least in part, on methods of crossing the pericardium and accessing the pericardial space, which are (usually) necessary precursors to accessing the epicardial surface of the heart.

For example, U.S. Patent No. 4,991,578 to Cohen describes a method and system for implanting self-anchoring epicardial defibrillation electrodes within the pericardial space. The system includes means for distending the pericardium from the heart by using suction or by injecting a small volume of fluid into the pericardium. A needle having a lumen therethrough is inserted from a subxyphoid or other percutaneous position into the body tissue until a tip thereof punctures the distended pericardium at a selected location. A guide wire is inserted into the pericardium through the lumen of the needle, and while the guide wire remains in the pericardial space, the needle is removed. A sheath is introduced over the guide wire, with the aid of a dilator, and inserted into the tissue until one end thereof is positioned within the pericardium. The defibrillation lead, with its electrode in a retracted position, is inserted through the sheath until the electrode is likewise positioned within the pericardium, whereupon the electrode is deployed in order to make contact with a large area of tissue within the pericardium.

Another example may be found in U.S. Patent No. 5,071,428 to Chin et al., which discloses a method and apparatus for providing intrapericardial access and inserting intrapericardial electrodes. Intrapericardial access is established by clamping the wall of the pericardium between elongate jaw elements carrying axially aligned tubular guides and passing a guide wire through the guides and the pericardial tissue therebetween. In the preferred embodiment, the jaw elements include interengageable ratchets for holding the elements in clamping engagement with the wall of the pericardium and aligned pointed extensions for piercing the pericardial tissue clamped between the elements. Further intrapericardial access is provided by an additional tubular guide carried by the jaw element intended to be disposed in the pericardium during placement of the guide wire.

Yet another example may be found in U.S. Patent No. 6,231,518 to Grabek et al., which discloses devices and methods for diagnosis and treatment of cardiac conditions through the pericardial space that are particularly suited for performing minimally invasive procedures from the surface of the heart including electrophysiology mapping and ablation, drug delivery, restenosis prevention, stent placement, etc. Preferred pericardial access devices use suction or mechanical grasping during access of the pericardium. The preferred devices provide for separating the parietal pericardium from the epicardial surface of the heart to reduce the chance of trauma to the heart wall during access of the pericardial space. Once the pericardial space is accessed, a material transport tube can be placed into the pericardial space for administering or removing materials from the pericardial space.

WO 03/066147 A1 discloses an access tube used for accessing an anatomic space, such as a pericardial space between the parietal and visceral pericardia. The access tube is advanced against the parietal pericardium and an anchor structure thereon embedded into the parietal pericardium. The access tube can then be used to separate the parietal and visceral pericardia and enlarge the pericardial space. After such enlargement, a needle or other access device can be introduced through the access tube into the pericardial space to provide access for a wide variety of purposes, including aspiration, infusion, and guidewire placement.

US 2003/0093104 A1 provides a method and apparatus for accessing the pericardial space to allow for stable short term or long term placement of a delivery catheter or cannula having its distal-most end fixed on the endocardial surface such that a pericardial access device including a dilator and a puncturing device may be advanced through the myocardium into the pericardial space. The catheter is provided with a distal fixation member that maintains the catheter position over the myocardial pericardial access created by the access device allowing various medical instruments to be passed through the pericardial access into the pericardial space. Alternatively, the catheter may be positioned against the septal wall to access a left heart chamber via a right heart chamber.

At least some of the above-described prior art techniques rely, at least in part, on the use of grasping or suction means to hold the pericardium in order to pull it away from the epicardial surface of the heart and pass a guide or other device therethrough. However, the disclosed grasping and suction means are not highly effective on fatty deposits, which are commonly present on the outer surface of the pericardium. Fatty deposits typically have little structural integrity and are easily delaminated from the pericardium, and therefore do not serve as a reliable means for holding the pericardium. Because fatty deposits are particularly common in older and/or overweight patients requiring cardiac therapy, these prior art techniques are not highly effective for a significant portion of the patient population.

### SUMMARY OF THE INVENTION

The present invention is defined by the appended claims. To address at least some of these needs, the present invention provides, in exemplary non-limiting embodiments, devices that may more dependably and consistently hold pericardial tissue to facilitate pericardial access and cardiac therapy. In an exemplary embodiment, a tissue grasping device is provided that may reliably hold pericardial tissue, even in the presence of fatty deposits. The tissue grasping portion may include a tissue penetrating tip, a tissue dilating distal section, a tissue retention neck and a tissue stop. When advanced into the pericardium, the tip may serve to create an opening (e.g., pierce, cut, etc.) in the pericardium, the distal section may serve to dilate the opening, the neck may serve to hold the tissue upon recoil of the dilated opening, and the stop may serve to limit further penetration once tissue is retained in the neck. The tissue grasping device may be used to facilitate pericardial access for a variety of therapeutic and/or diagnostic applications as will be described in more detail hereinafter.

According to an example, the disclosure provides a method for accessing the pericardial space of the heart. The method may comprise, from a remote location, inserting a portion of an access device through the pericardium such that the portion automatically is inserted to a predetermined depth beyond the pericardium. After inserting the portion through the pericardium, the method may further comprise separating the pericardium from the epicardium via the inserted portion of the access device.

In yet another exemplary aspect, a method for separating a first tissue layer from a second tissue layer that is less fibrous than the first tissue layer, so as to provide access to the space between the tissue layers may comprise inserting a portion of an access device through the first tissue layer such that the portion automatically is inserted to a predetermined depth beyond the first tissue layer. The method further may comprise engaging the first tissue layer with the inserted portion of the device and separating the first tissue layer from the second tissue layer by moving the inserted portion in a proximal direction substantially opposite to the direction of insertion.

Yet another exemplary aspect of the invention includes an apparatus for accessing a space between a first layer of tissue and a second, adjacent layer of tissue that is less fibrous than the first layer of tissue. The device may comprise a shaft having a distal portion, wherein the distal portion is configured to be inserted at least through the first tissue layer when the shaft is advanced in a first insertion direction. The distal portion may be further configured to engage with the first tissue layer and to separate the first tissue layer from the second tissue layer when the distal portion is moved in a second direction substantially opposite to the first direction.

A further exemplary aspect includes an apparatus for accessing the pericardial space of the heart to perform a medical procedure. The apparatus may comprise a distal portion being configured to be automatically inserted through the pericardium to a predetermined depth beyond the pericardium and to separate the pericardium from the epicardium. The apparatus may further be configured to provide access to the pericardial space from a location remote from the pericardial space.

According to another exemplary aspect, the invention includes an apparatus for delivering medical devices to the pericardial space of a heart. The apparatus may comprise a dilator shaft having a distal end, a proximal end, and a lumen configured to receive at least one medical device. The dilator shaft may have an expanded region proximate the distal end of the shaft, the expanded region being configured to be positioned in the pericardial space and to engage the pericardium while the at least one medical device is advanced through the lumen and into the pericardial space.

### BRIEF DESCRIPTION OF THE DRAWINGS

Aside from the structural and procedural arrangements set forth above, the invention could include a number of other arrangements, such as those explained hereinafter. It is to be understood that both the foregoing summary and the following detailed description are exemplary. The accompanying drawings are included to provide a further understanding of the invention and are incorporated in and constitute a part of this specification. Together with the following detailed description, the drawings illustrate exemplary embodiments and serve to explain certain principles. In the drawings,
Figures 1A and 1B are schematic illustrations of pericardial access devices;
Figures 2A - 2H are schematic illustrations showing the device of Figure 1A in use;
Figures 3A - 3C show a pericardial access device similar to the device of Figure 1A but with more detail;
Figure 3D shows a guide wire for use in the device of Figures 3A - 3C;
Figure 4 is an anatomical illustration showing a sub-xyphoid approach for using a pericardial access device;
Figure 5 is a block diagram showing how a pericardial access device may be used;
Figure 6 is a block diagram showing in more detail how a pericardial access device may be used;
Figures 7 - 13 are schematic illustrations of various alternative pericardial access devices; and
Figures 14 - 15 are schematic illustrations of access supplementation devices.

### DETAILED DESCRIPTION OF EXEMPLARY EMBODIMENTS

The following detailed description should be read with reference to the drawings in which similar elements in different drawings are numbered the same. The drawings, which are not necessarily to scale, depict illustrative embodiments and are not intended to limit the scope of the invention.

The devices and methods described herein are discussed herein with reference to the human heart H, but may be equally applied to other 'animal hearts not specifically mentioned herein. For purposes of discussion and illustration, several anatomical features may be labeled as follows: dermal layer DL; sternum ST; xiphoid XPH; diaphragm DPH; heart wall HW; pericardium P; pericardial space PS; and fatty deposit F.

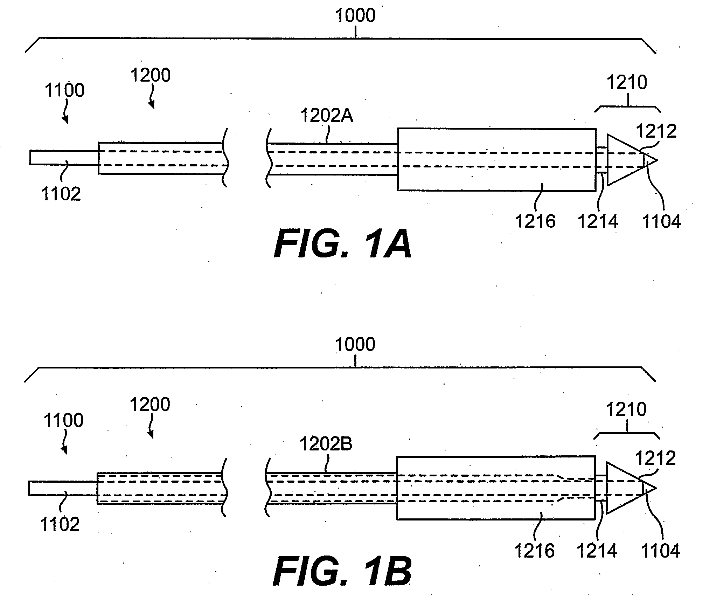

With reference to Figures 1A and 1B, schematic embodiments of a pericardial access device 1000 are shown. Pericardial access device 1000 may be used to facilitate a variety of cardiac therapies and diagnostics as will be described in more detail hereinafter. Generally, the access device 1000 provides for less invasive surgical access from a point outside the patient's body, through a transthoracic port (e.g., subxyphoid or intercostal) to the pericardial space around the patient's heart, as will be described in more detail hereinafter. Alternative access devices and approaches are described in U.S. Published Patent Application No. 2004/0148019 A1 to Vidlund et al., all of which may be utilized in one form or another to facilitate the therapeutic and diagnostic techniques described hereinafter.

With continued reference to Figures 1A and 1B, pericardial access device 1000 includes a stylet member 1100 and a trocar member 1200. Stylet member 1100 is removably insertable into trocar member 1200. The trocar member 1200 includes an elongate shaft 1202 and a tissue grasping portion 1210 that, together with the tip of the stylet member 1100, assists in piercing and retaining the pericardial sac such that it may be pulled away from the heart to enlarge the pericardial space. In Figure 1A, the shaft 1202A includes a lumen (shown by dashed line) extending therethrough that is sized to accommodate the stylet 1100 and the guide wire 1300 (shown in subsequent Figures) in sequence. In Figure 1B, the shaft 1202B includes a lumen (shown by dashed line) extending therethrough that is sized to accommodate the stylet 1100 and the guide wire 1300 side-by-side. More specifically, in the embodiment of Figure 1B, the proximal portion of the lumen in the shaft 1202B may have a relatively larger size to slidably accommodate the stylet 1100 and the guide wire 1300 side-by-side, and the distal end of the lumen may have a relatively smaller size to accommodate the stylet 1100 and the guide wire 1300 individually.

Once the pericardial sac is pierced and retained by the tissue grasping portion 1210, and the pericardial sac is pulled away from the heart to enlarge the pericardial space, the stylet member 1100 may be removed from (embodiment of Figure 1A) or retracted into (embodiment of Figure 1B) the trocar member 1200 and a guide wire 1300 may be advanced in its place. With the guide wire extending through the trocar member 1200 and into the pericardial space, the trocar member 1200 may be removed leaving the guide wire in place. The guide wire thus provides pericardial access from a remote site outside the patient's body and may be used to guide and advance other devices into the pericardial space as described herein.

The tissue grasping portion 1210 includes a tissue penetrating tip 1104, a tissue dilating distal section 1212, a tissue retention neck 1214 and a tissue stop 1216. When advanced into the pericardium, the tip 1104 may serve to create an opening (e.g., pierce, cut, etc.) in the pericardium, the distal section 1212 may serve to dilate the opening, the neck 1214 may serve to hold the tissue upon recoil of the dilated opening, and the stop 1216 may serve to limit further penetration once tissue is retained in the neck.

To further illustrate the operation of the tissue grasping portion 1210, it is helpful to consider the environment in which it is particularly suited for use. The pericardial space PS is defined between the pericardium P (a.k.a., pericardial sac) and the outside (epicardial) surface of the heart HW. The pericardial sac is very close to (and often in intimate contact with) the epicardial surface of the heart. Therefore, it is helpful to separate the pericardium from the epicardium to provide ready and safe access to the pericardial space. Although separating the pericardium from the epicardium may be readily accomplished using open surgical techniques, it is far more difficult to do so using remote access techniques (e.g., endoscopic, transthorascopic, percutaneous, etc.). To delineate between the epicardial and pericardial layers, the tissue grasping portion 1210 selectively penetrates the pericardial tissue to a limited extent when advanced, and holds onto pericardial tissue when retracted.

More specifically, the tissue grasping portion 1210 is configured to hold onto fibrous tissue such as the pericardium, while not holding onto other less fibrous tissues such as the heart wall (epicardium, myocardium, and endocardium) and surrounding fatty tissues. The tissue grasping portion 1210 is also configured to readily pass through fibrous tissue to a predetermined, limited depth. With this arrangement, the tissue grasping portion 1210 may be advanced to penetrate various layers of fibrous and less-fibrous tissue, stop at a predetermined depth when a fibrous tissue layer is penetrated, and upon retraction, grasp onto the fibrous tissue layer (and not the other less-fibrous layers) to pull the fibrous layer away from the adjacent less-fibrous layer. For example, the pericardial access device 1000 may be inserted from a point outside the cardiac space toward the heart, automatically stop when the pericardium is penetrated to a prescribed depth, and selectively hold onto the pericardium when retracted to pull the pericardium away from the epicardial surface, thereby increasing the pericardial space and providing ready access thereto.

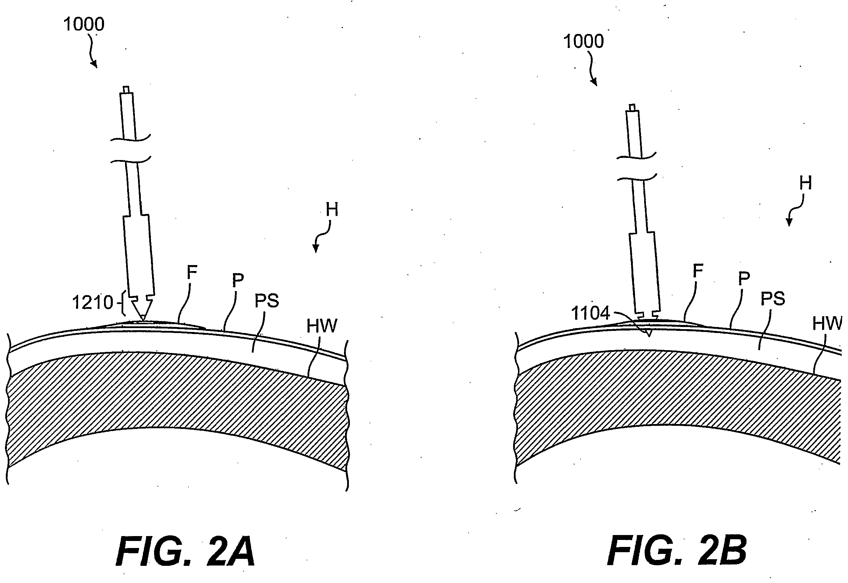

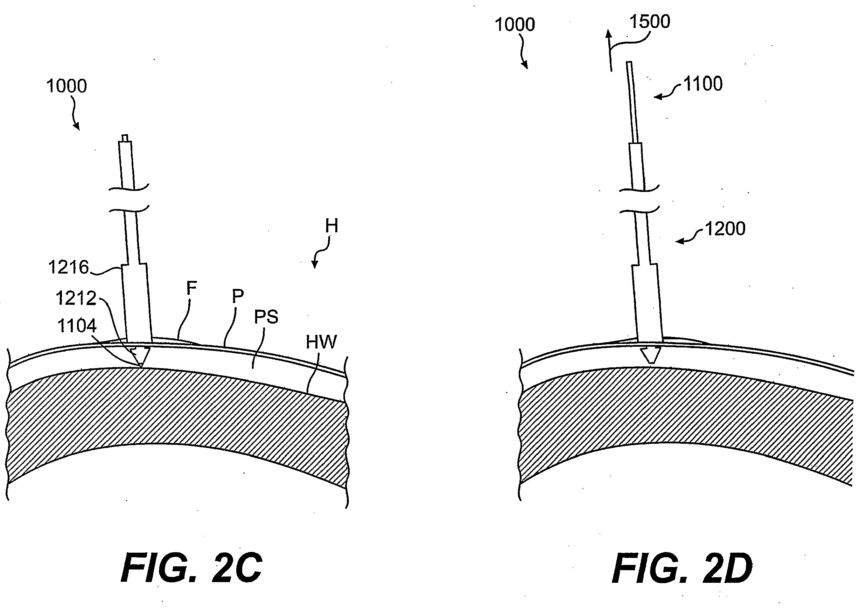

With reference to Figures 2A - 2H, the functional operation of the pericardial access device 1000, and in particular tissue grasping portion 1210, may be appreciated in more detail. The access device is advanced (e.g., from a sub-xyphoid or intercostal approach) toward the heart H until the distal end thereof is proximate or engages the pericardium P or fatty deposits F residing on the outside of the pericardium as shown in Figure 2A. The device 1000 is then advanced further such that the tip 1104 penetrates and creates an initial opening in the fatty deposit F and the pericardium P until it resides in the pericardial space PS as shown in Figure 2B. As the device 1000 is advanced further, the distal section 1212 (obstructed and thus not labeled in Figure 2B, but visible in Figures 1A and 1B) elastically dilates the pericardium P to increase the size of the initial opening created by the tip 1104. After the distal section 1212 passes through the pericardial layer, the pericardium elastically recoils about the neck 1214 (obstructed and thus not labeled in Figure 2C, but visible in Figures 1A and 1B) and is retained therein as shown in Figure 2C. The stop (or shoulder) 1216 abuts the recoiled pericardium and prevents further advancement of the device therethrough, and thereby limits excessive penetration or prevents penetration of the tip 1104 into the heart wall HW.

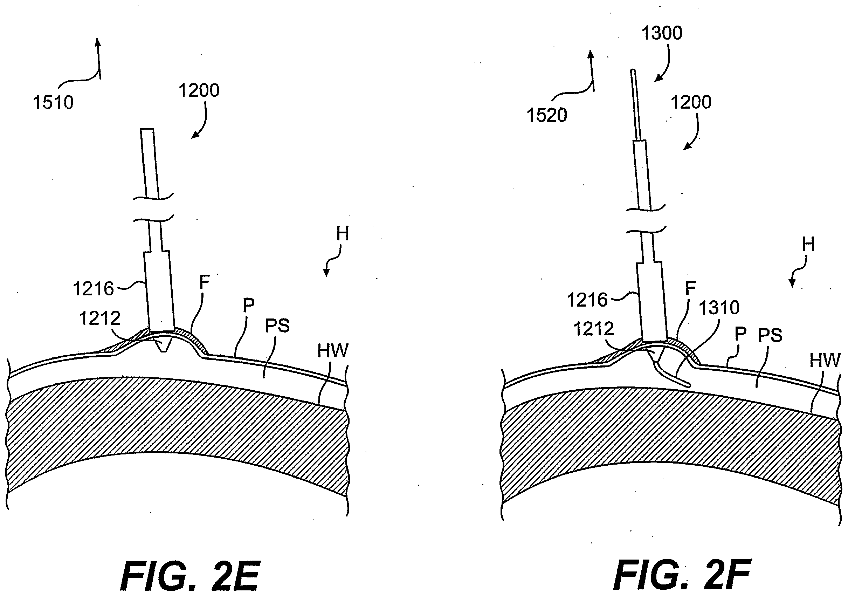

With the pericardium P retained in the neck 1214 of the tissue grasping portion 1210, the stylet 1100 is removed proximally from the internal lumen of the trocar 1200 as indicated by arrow 1500 shown in Figure 2D. Before or after the stylet 1100 is removed, the trocar 1200 may be pulled proximally as indicated by arrow 1510 shown in Figure 2E. By pulling proximally on the trocar 1200 with the pericardium P retained in the neck 1214 of tissue grasping portion 1210, the pericardium P is pulled away from the heart wall HW to increase the space therebetween and allow safe passage into the pericardial space PS of devices subsequently advanced through the trocar 1200. For example, after the stylet 1100 has been removed from the trocar 1200, a guide wire 1300 may be advanced distally through the internal lumen of the trocar 1200 as indicated by arrow 1520 in Figure 2F. The guide wire 1300 is advanced through the trocar 1200 until the distal end portion 1310 thereof resides in the pericardial space.

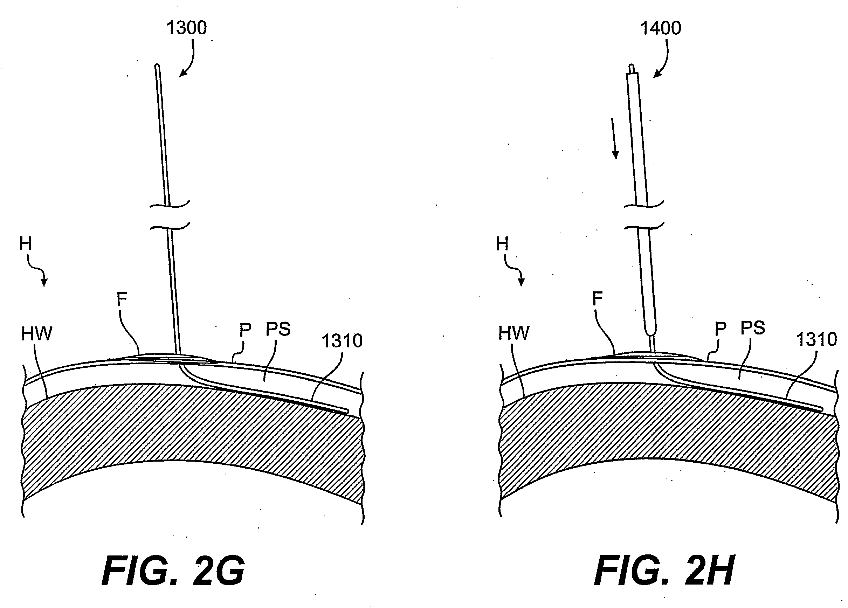

With the guide wire 1300 extending through the trocar 1200 and the distal end portion 1310 of the guide wire 1300 residing in the pericardial space, the guide wire 1300 may be further advanced into the pericardial space PS to avoid accidental dislodgement therefrom as the trocar 1200 is removed, leaving the guide wire 1300 in place as shown in Figure 2G. With the guide wire 1300 defining a path from a remote site outside the patient's body to the pericardial space PS, subsequent devices such as dilator 1400 may be readily advanced thereover to supplement access (e.g., enlarge luminal size, retain pericardium, etc.) to the pericardial space PS as shown in Figure 2H. Once pericardial access is established, therapeutic and/or diagnostic devices may be inserted therein as will be described in more detail hereinafter.

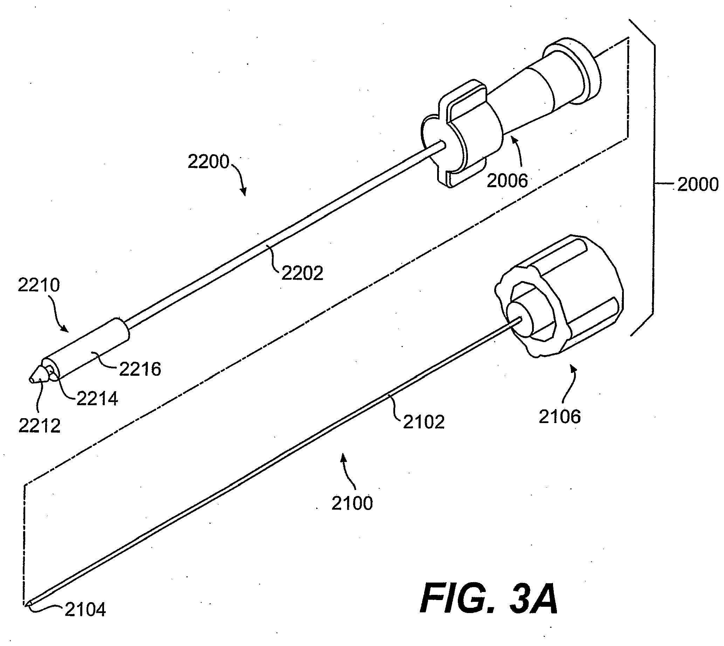

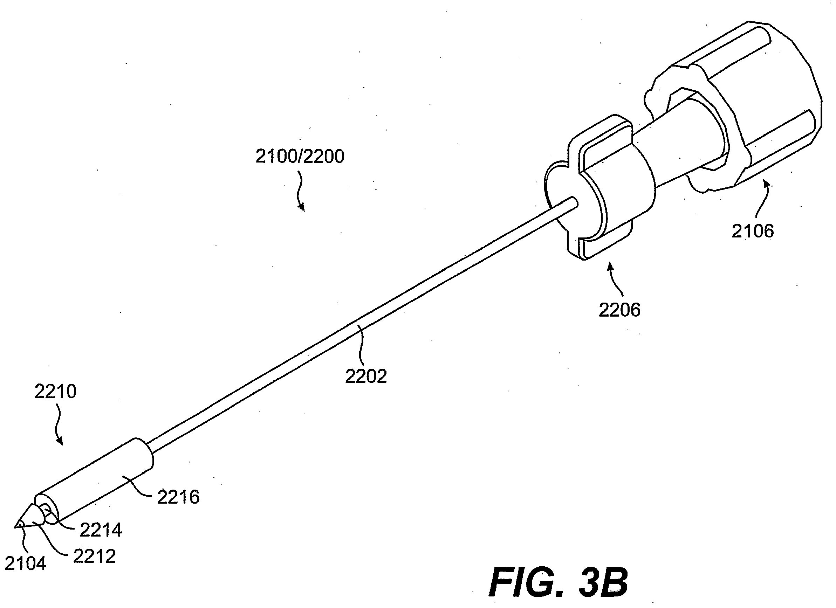

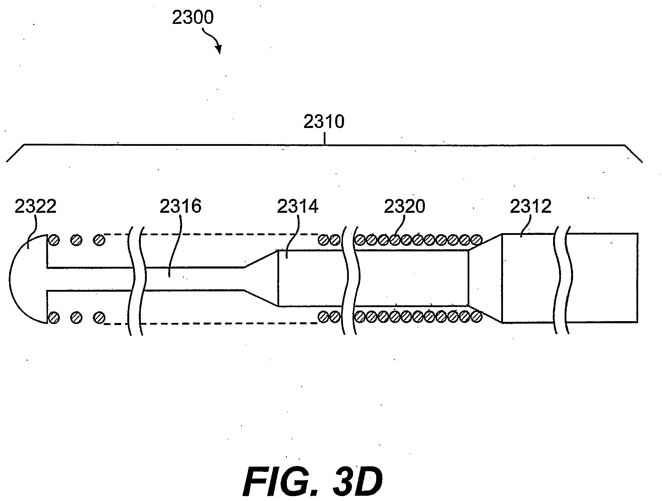

'With reference to Figures 3A - 3C, a more detailed embodiment of an access device 2000 is shown. With specific reference to Figure 3A, the alternative access device 2000 includes a stylet member 2100 and a trocar member 2200. Stylet member 2100 is removably insertable into trocar member 2200 as shown in Figure 3B. The trocar member 2200 includes a tissue grasping portion 2210 that, together with the tip of the stylet member 2100, assists in piercing and retaining the pericardial sac such that it may be pulled away from the heart to enlarge the pericardial space. Once this is accomplished, the stylet member 2100 may be removed from the trocar member 2200 and a guide wire 2300, as shown in Figure 3D, may be inserted its place. With the guide wire 2300 extending through the trocar member 2200 and into the pericardial space, the trocar member 2200 may be removed leaving the guide wire 2300 in place. The guide wire 2300 thus provides pericardial access from a remote site and may be used to guide and advance delivery devices as described herein.

Stylet member 2100 includes an elongate shaft 2102 having a tissue piercing distal tip 2104 and a proximal hub 2106. Trocar member 2200 includes an elongate hollow shaft 2202, a distally disposed tissue grasping portion 2210 and a proximally disposed hub 2206. The shaft 2202 may comprise a stainless steel hypotube with a lumen extending therethrough. The lumen in the trocar member 2200 may extend through the hub 2206, hollow shaft 2202 and distal tissue grasping portion 2210. The elongate shaft 2102 of the stylet member 2100 is insertable into the lumen extending through the trocar member 2200 such that the distal tip 2104 of the stylet device 2100 protrudes from the distal end of the tissue grasping portion 2210 when the proximal hub 2106 of the stylet member 2100 engages and locks with the proximal hub 2206 of the trocar member 2200 as best seen in Figure 3B. When assembled, the tip 2104 functions integrally with the tissue grasping portion 2210 and may be considered a part thereof.

The tip 2104 of the stylet member 2100 is configured to pierce tissue, particularly fibrous tissue such as the pericardium surrounding the heart, and less fibrous tissue such as the fatty tissues disposed on the exterior of the pericardium. The tip 2104 may be conical with a sharp apex, semi-conical with one or more sharpened edges, or any other geometry suitable for piercing fibrous tissue. Proximal of the apex, the shape of the tip 2104 may be configured to dilate fibrous tissue, such that once the apex pierces the fibrous layer, the tip serves to dilate (as opposed to cut) the hole initiated by the apex. For example, proximal of the apex, the tip 2104 may be circular in cross-section to minimize propagation of the hole initiated by the apex.

A smooth transition may be provided between the tip 2104 of the stylet 2100 and the tissue dilating distal section 2212 of the tissue grasping portion 2210 such that the distal section 2212 continues to dilate the tissue pierced by the apex of the tip 2104. The distal section 2212 may be the same or similar geometry (e.g., conical with a circular cross-section) as the tip 2104 proximal of the apex. A neck 2214 may be provided proximal of the distal section 2212, the profile (e.g., diameter) of which may be selected to allow the fibrous tissue to elastically recoil and resist withdrawal. A shoulder or stop 2216 may be provided proximal of the neck 2214, the profile (e.g., diameter) of which may be selected to limit or stop penetration of the tip 2104 once the shoulder 2216 engages fibrous tissue. Thus, the tip 2104 and distal section 2212 may be configured to penetrate and dilate fibrous tissue, the neck 2214 may be configured to permit elastic recoil of the fibrous tissue and resist withdrawal therefrom, and the shoulder 2216 may be configured to stop penetration through fibrous tissue.

Various sizes and geometries of the aforementioned components are contemplated consistent with the teachings herein. The size and geometry of the tip 2104, and in particular the apex of the tip 2104, may be selected to initially penetrate fibrous tissue (e.g., pericardial tissue) and less-fibrous tissue (e.g., fatty tissue, epicardial tissue, myocardial tissue, etc.). The size and geometry of the tip 2104 proximal of the apex, and the size and geometry of the distal section 2212 may be selected to elastically dilate (but not over-dilate) fibrous tissue initially penetrated by the apex of the tip 2104. The degree of elastic dilation of the fibrous tissue may be sufficiently high to provide for elastic recoil around the neck 2214, but not so high as to cause plastic dilation or tearing of the fibrous tissue. The size and geometry of the neck 2214 may be selected such that the fibrous tissue elastically recoils sufficiently to create a relatively high withdrawal force permitting the fibrous tissue layer to be pulled away from adjacent less-fibrous layers without tearing the fibrous tissue layer. The size and geometry of the shoulder 2216 may be selected such that further penetration is prohibited once the shoulder 2216 engages fibrous tissue.

Taking advantage of the fact that fibrous tissue is relatively tough, tends to elastically deform and tends not to tear, whereas less-fibrous or non-fibrous tissue is weaker and tends to plastically deform or tear, the combination of sizes and geometries of the tip 2104, distal portion 2212, neck 2214 and shoulder 2216 may be selected to advance and penetrate through both fibrous and less-fibrous tissue, stop penetration once fibrous tissue is encountered, and grasp the fibrous tissue (while releasing the less-fibrous tissue) upon retraction. As such, the size and geometry of the aforementioned elements may be selected as a function of the characteristics of the tissue layers being separated. In particular, the dimensions and geometries may be chosen to selectively secure (e.g., hold or grasp) tissue of a relatively higher degree of fibrousness or toughness, and release (e.g., not hold or grasp) tissue of a relatively lower degree of fibrousness or toughness.

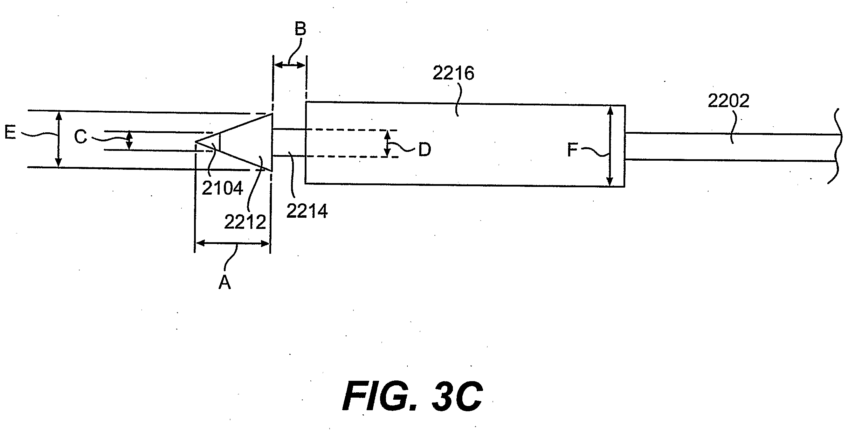

For selective securing of the pericardium, Figure 3C and the following Table 1 provides example working dimensions by way of illustration, not limitation. Those skilled in the art will recognize that depending on the tissue layers being separated, these dimensions may be modified according to the teachings herein.

**Table 1**

| Dimension | Example Range | Working Example #1 | Working Example #2 |
|---|---|---|---|
| A | 0.063 - 0.125" | 0.125" | 0.063" |
| B | 0.020 - 0.060" | 0.040" | 0.020" |
| C | 0.011 - 0.020" | 0.020" | 0.011" |
| D | 0.032 - 0.065" | 0.032" | 0.020" |
| E | 0.032 - 0.065" | 0.065" | 0.032" |
| F | 0.080 - 0.100" | 0.090" | 0.080" |

With reference to Figure 3C and the working examples in Table 1, a number of general observations and statements may be made. For example, after the pericardium is initially pierced by the apex of tip 2104, dimensions A and E are important to achieve the desired amount of elastic pericardial dilation without tearing. Generally speaking, the more pericardial tearing that occurs, the less pericardial retention is achieved. Thus, the larger dimension E is, the longer dimension A may need to be to cause pericardial dilation and minimize tearing. Also, the greater dimension A is relative to E, the lower the force that is required to pierce the pericardium and subsequently dilate it, which may be desirable in some instances. After the pericardium is dilated to the desired degree, the difference between dimensions D and E are important to achieve the desired amount of pericardial retention. To this end, the step from the distal portion 2212 to the neck portion 2214 may be defined as dimension (E - D). Generally speaking, the more elastic pericardial dilation that occurs, the smaller step (E - D) may be to achieve adequate retention. Note also that the depth of tissue penetration is generally governed by the sum of dimensions A and B. While B must be sufficiently wide to accommodate the pericardial layer, dimension A may be adjusted to reduce penetration too far beyond the pericardial layer.

From the foregoing, it is apparent that the tissue grasping portion 2210 together with the tip 2104 of the stylet member 2100 assist in piercing and retaining the pericardial sac such that it may be pulled away from the heart to enlarge the pericardial space. Once this is accomplished, the stylet member 2100 may be removed from the trocar member 2200 and a guide wire 2300, as shown in Figure 3D, may be inserted in its place. Alternatively, the proximal portion of the lumen in the shaft 2202 of the trocar member 2200 may have a relatively larger size to slidably accommodate the stylet 2100 and the guide wire 2300 side-by-side, and the distal end of the lumen may have a relatively smaller size to accommodate the stylet 2100 and the guide wire 2300 individually, thus allowing the stylet 2100 to be pulled proximally but not removed from the trocar 2200 and the guide wire 2300 to be advanced distally in its place. Although a wide variety of guide wire designs may be employed for this purpose, the guide wire design illustrated in Figure 3D has some advantages, particularly when used in combination with trocar member 2200.

With continued reference to Figure 3D, a distal portion of the guide wire 2300 is shown in longitudinal cross-section. Guide wire 2300 includes an elongate shaft 2310 having a proximal end and a distal end. The flexibility of the shaft 2310 increases from its proximal end to its distal end, which may be accomplished by providing reduced diameter or changes in cross section along its length. In the illustrated embodiment, the shaft 2310 of the guide wire 2300 includes a relatively stiff proximal core portion 2312 having a circular cross section, a relatively flexible middle portion 2314 having a rectangular (ribbon-like) cross section, and a highly flexible distal end portion 2316 having a rectangular (ribbon-like) cross section. A radiopaque coil 2320 may be wound around the middle portion 2314 and distal portion 2316, with a proximal end connected to the distal end of the proximal core portion 2312, and a distal end terminating in a distal weld ball 2322 connected to the distal end portion 2316. The distal turns of the coil 2320 may be spaced apart to reduce column strength and increase flexibility as will be discussed in more detail hereinafter.

The guide wire 2300 may be formed of conventional materials using conventional techniques, and may have conventional dimensions except as may be noted herein. The following dimensions are given by way of example, not limitation. The guide wire 2300 may have a diametric profile of about 0.018 inches, for example, or other dimension sized to fit through trocar 2200. In the illustrated embodiment, the proximal core portion 2312 may have a diameter of about 0.018 inches, and the outer profile of the coil 2320 may also have a diameter of about 0.018 inches. The middle portion 2314 may be about 0.010 x 0.002 inches in cross section, and the distal portion 2316 may be about 0.002 x 0.004 inches in cross section and about 1.0 inches in length. The guide wire 2300 may have an overall length of about 44.0 inches, for example, or other dimension sized to extend through and beyond the ends of the trocar 2200 and to provide sufficient length for subsequent devices (e.g., sheaths, dilators, balloon catheters, etc.) to be advanced over the wire 2300. The length of the highly flexible portion(s) of the guide wire 2300 may be selected to be longer than the trocar 2200 such that the guide wire 2300 buckles at the proximal end thereof at a lower force than is required to cause the distal end thereof to penetrate into the epicardial surface of the heart wall.

The middle 2314 and distal 2316 portions of the guide wire 23 00 form an atraumatic section. The middle portion 2314 is highly flexible due to its ribbon-like cross-section and relatively small dimensions. The distal portion 2316 has both high flexibility (due to its ribbon-like cross-section and relatively small dimensions) and low buckle strength (due to the spacing of coil turns). Thus, the middle 2314 and distal 2316 portions are rendered atraumatic. This is particularly true for the distal portion 2316 which is the first portion of the guide wire 2300 to extend beyond the distal end of the trocar 2200 when the guide wire is fully inserted therein. The combination of the loosely spaced coils 2320 and the highly flexible ribbon 2316 allows the distal end of the guide wire to deflect laterally when it extends out of the distal end of the trocar and engages the heart wall. Because the buckle strength of the highly flexible atraumatic distal portion is less than the force required to penetrate the heart wall (as may occur with stiffer conventional wires), the risk of the guide wire 2300 inadvertently penetrating into the heart wall when advanced through the distal end of the trocar 2200 is minimized.

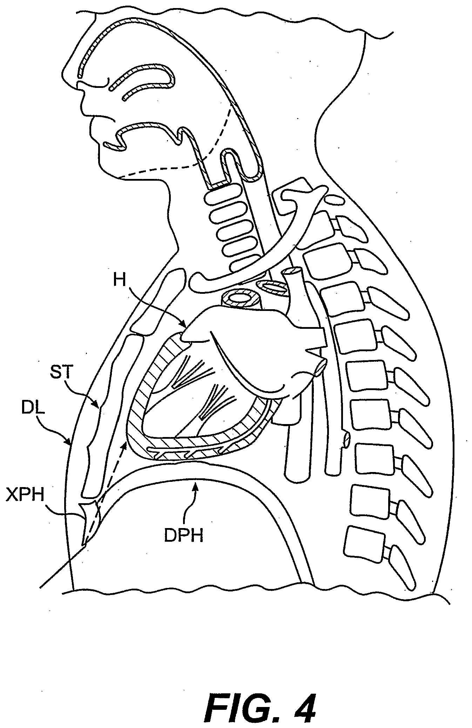

The pericardial access devices 1000, 2000 described hereinbefore are particularly suitable for a transthoracic anterior approach as shown in Figure 4 with a dashed line and a distal arrow. The approach utilized for devices 1000, 2000 may comprise a subxiphoid approach as shown. However, a lateral or posterior approach may utilize similar devices 1000, 2000 and techniques to access the pericardial space from the side or back between the ribs (intercostal space).

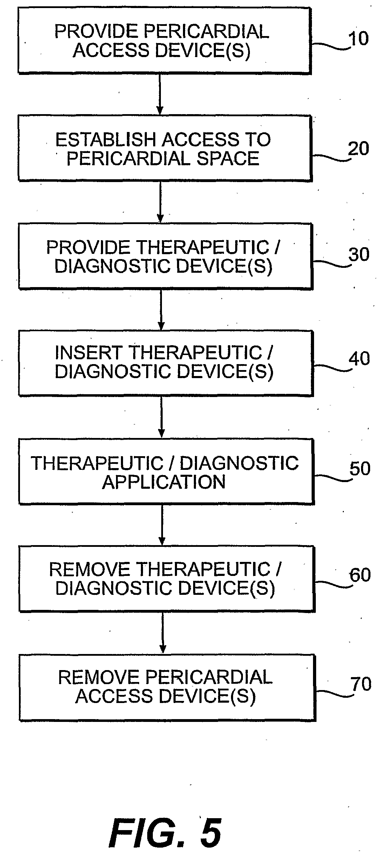

A general method for using access devices 1000, 2000 is illustrated by block diagram in Figure 5. It is noted that the methods described herein do not form part of the invention but are useful for understanding the invention. As indicated by block 10, the method includes the initial step of providing a suitable access device 1000, 2000 such as those described previously. As indicated by block 20, access to the pericardial space may then be established as generally described previously and as described in more detail hereinafter. As indicated by block 30, therapeutic and/or diagnostic device(s) may then be provided depending on the particular clinical application. A variety of clinical applications are enabled by this pericardial access method, non-limiting and non-exhaustive examples of which include: epicardial lead placement and pacing, cardiac repair, valve repair, left atrial appendage occlusion, pulmonary vein occlusion, cardiac ablation, drug delivery, cardiac tamponade relief, cardiac biopsy, and minimally invasive CABG. Each of the foregoing clinical applications involves a particular set of therapeutic/diagnostic devices and associated delivery tools, non-limiting and non-exhaustive examples of which include: epicardial pacing leads, cardiac restraint devices, valve repair devices, left atrial appendage occlusion devices, pulmonary vein occlusion devices, cardiac ablation systems, drug delivery catheters, cardiac tamponade relief devices, biopsy devices, minimally invasive CABG devices, etc., together with their associated delivery tool(s). As indicated by block 40, the delivery device(s) and the associated therapeutic/diagnostic device(s) may be inserted over or through the access means (e.g., guide wire or sheath, depending on the application) and into the pericardial space PS. The therapeutic or diagnostic application may then be performed as indicated by block 50. After the therapeutic or diagnostic session is complete, the devices may be removed as indicated by blocks 60 and 70. Examples of suitable valve repair devices (e.g., devices for improving valve function) and their corresponding methods of use are disclosed in U.S. Patent Application No. 10/704,145 (U.S. Published Patent Application No. 2004/0148020 A1), filed on a date even herewith, entitled DEVICES AND METHODS FOR HEART VALVE TREATMENT to Vidlund et al.

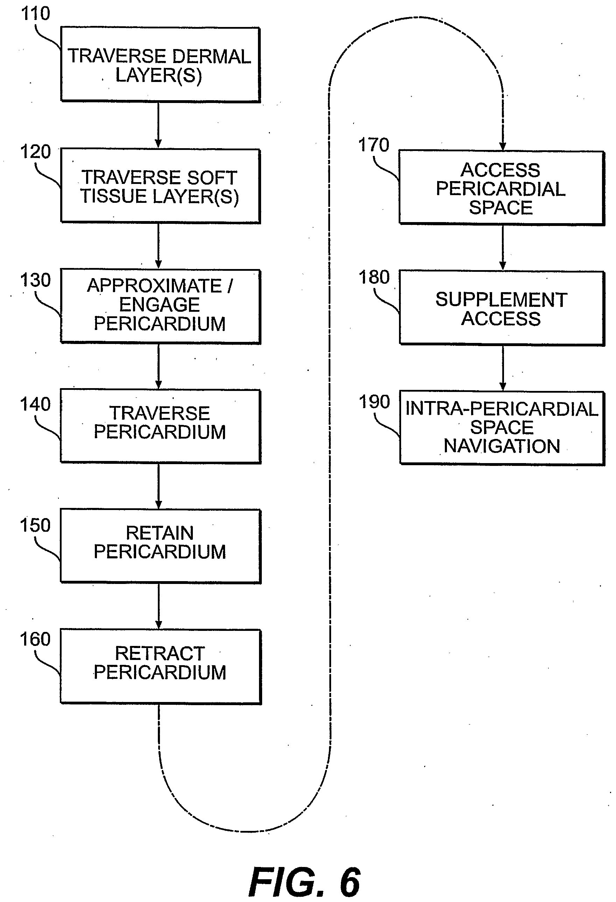

The method of establishing pericardial access as indicated by block 20 in Figure 5 may be broken down into more detail as illustrated in Figure 6. The breakdown shown in Figure 6 is given by way of example, not limitation, to better understand some of the functional aspects of the devices and methods described elsewhere herein. In the illustrated example, pericardial access includes the following sub-steps: dermal traversal 110; soft tissue traversal 120; pericardial engagement/approximation 130; pericardial traversal 140; pericardial retention 150; pericardial retraction 160; pericardial space access 170; access supplementation 180; and intra-pericardial space navigation 190. These steps may be taken alone or in a variety of combinations, divisions or repetitions, and the order may be modified as well.

The sub-steps of percutaneous traversal 110, soft tissue traversal 120, and pericardial engagement/approximation 130 may be accomplished using conventional tools and techniques modified for this particular application. In a percutaneous method, a needle and wire, and/or blunt dilator and/or introducer may be used to pierce and dilate dermal and soft tissue layers. Alternatively, in a surgical method, a blade and/or coring device and/or cautery device may be used to cut or bore through dermal and soft tissue layers. As a further alternative, a combination of theses tools and methods may be employed for a hybrid percutaneous/surgical methodology. For example, as generally shown in Figure 4, a small incision may be made in the dermal layers and sub-dermal soft tissue layers just below the xyphoid in the direction of the cardiac space just above the diaphragm (to avoid accessing the pleural space and thus eliminating the need for venting). An introducer sheath (e.g., 8F) and dilator may be inserted through the incised area in a direction toward the inferior-anterior side of the pericardial space, generally coplanar with the annulus of the mitral valve. The desired position of the distal end of the introducer (which may be radiopaque) may be confirmed and/or adjusted using fluoroscopic techniques, and once the introducer is in the desired position, the dilator may be removed therefrom. Thus, the introducer sheath extends across the dermal and soft tissue layers and the distal end thereof engages the pericardial sac or resides adjacent thereto.

The sub-steps of pericardial traversal 140, pericardial retention 150, pericardial retraction 160, and pericardial space access 170 may be accomplished using the system described with reference to Figures 3A - 3D. For example, with the introducer sheath extending into the chest cavity and its distal end residing adjacent the pericardial sac, and with the dilator having been removed, the access device 2000 (stylet member 2100 and trocar member 2200 assembled) may be inserted into the introducer until the distal tip thereof engages the pericardium. The position of the distal end of the access device (which may be radiopaque) may be confirmed and/or adjusted using fluoroscopic techniques (e.g., AP and lateral views) to ensure the proper pericardial access point and avoid critical coronary structures (e.g., coronary arteries). To further ensure that critical coronary structures such as arteries, veins, etc. are not in the direct path of the access device 2000, fluoroscopic techniques may be employed to illuminate the coronary vasculature and visualize the anticipated path of the access device 2000 relative thereto.

With tactile feedback and fluoroscopic visualization guiding the physician, the access device 2000 may be further advanced until the tip penetrates the pericardial sac and the shoulder engages the outside of the pericardium to stop further penetration. Once the pericardium is penetrated and the shoulder abuts the outside of the pericardial sac, the pericardial layer resides within the neck recess of the access device and is retained therein. The stylet member 2100 may be removed from the trocar member 2200, and a guide wire 2300 may be inserted in its place. While applying gentle proximal traction to the trocar member 2200 to pull the pericardium away from the heart wall, the guide wire 2300 may be advanced until its distal atraumatic end extends beyond the distal end of the trocar 2200 and into the pericardial space. With the guide wire 2300 defining a path extending from a location outside the body, into and partially through the chest cavity, and into the pericardial space, the trocar 2200 and the introducer sheath may be removed therefrom.

The sub-step of access supplementation 180 may be accomplished using additional guides, sheaths, dilators, guide wires and/or by a balloon catheter or mechanical dilator advanced over the guide wire. For example, the balloon catheter or dilator may be used to enlarge the size of the hole in the pericardium. A guide catheter (e.g., 6F) may then be advanced over the guide wire into the pericardial space, and the relatively small (0.018 inch diameter) guide wire may be replaced with a relative large (0.035 inch diameter) guide wire. A larger introducer sheath and dilator may then be advanced over the larger guide wire, and the dilator and guide wire may then be removed from the sheath. Thus, the relatively large bore introducer defines a path extending from a location outside the body, into and partially through the chest cavity, and into the pericardial space, thus providing a path for subsequent therapeutic/diagnostic devices.

The sub-step of intra-pericardial space navigation 190 may be accomplished in part by curves provided in the introducer sheath and/or curves provided in the delivery system associated with the particular therapeutic/diagnostic device(s) utilized. However, the extent of intra-pericardial space navigation may be minimized by the appropriate access approach as shown in Figure 4. For example, a desirable access approach results in an introducer extending across the right ventricle and toward the left ventricle, with the curve of the introducer sheath directed toward the left ventricle, which is a common destination site for many therapeutic and diagnostic applications.

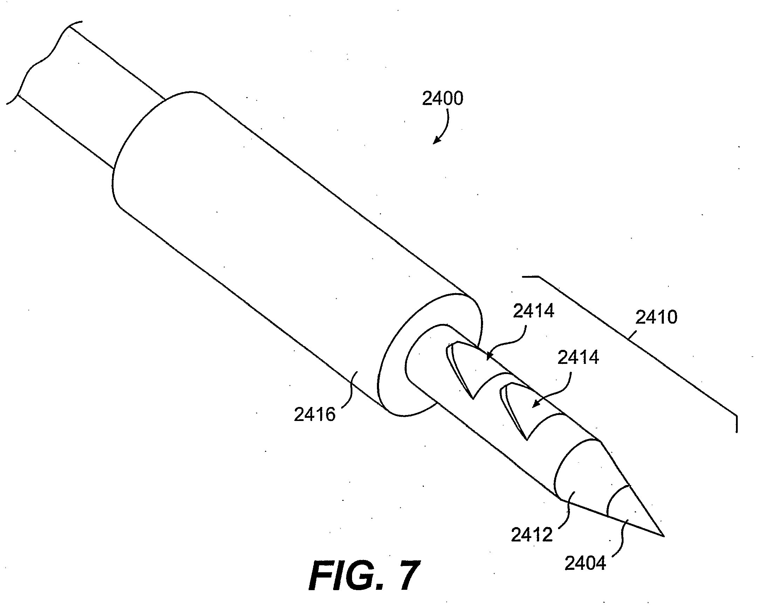

With reference to Figure 7 (not forming part of the present invention), an alternative pericardial access device 2400 is shown. Access device 2400 includes a different tissue grasping mechanism 2410, but may be used with the same stylet 2100 as described previously including tip 2404. Tissue grasping portion 2410 includes a tissue dilating distal section 2412 and a proximal stop or shoulder 2416 serving the same or similar functions as described previously. Tissue grasping portion 2410 also includes one or more protrusions 2414 that may comprise barbs, ridges, tapered edges (as shown), etc., or may alternatively comprise indentations. The protrusions 2414 may be formed, for example, by partially slicing into the wall of a polymeric (e.g., PEEK) tube and bending the sliced portion outward. The protrusions 2414 may serve to further elastically dilate tissue and retain the tissue upon recoil, similar to the function of the neck described previously.

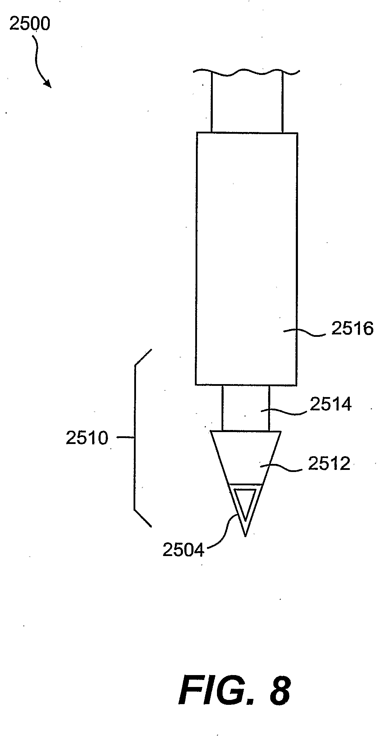

With reference to Figure 8, an alternative pericardial access device 2500 is shown. Access device 2500 includes a different tissue grasping mechanism 2510, and may be used with the same stylet 2100 as described previously with a modified tip 2504. Tip 2504 includes one or more sharpened edges to reduce the force required to pierce through the pericardium (and other tissues). This may be advantageously employed to penetrate through the pericardium without pushing the pericardium against the heart wall, thus reducing the risk of piercing coronary vasculature. The remaining elements of the tissue grasping portion 2510 may be the same or similar as described previously, including tissue dilating distal section 2512, neck 2514 and stop or shoulder 2516.

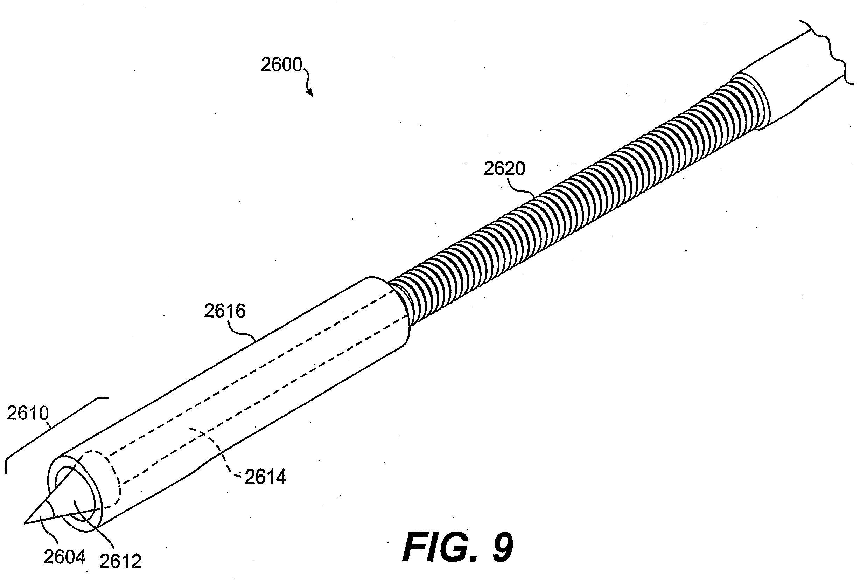

With reference to Figure 9 (not forming part of the present invention), an alternative pericardial access device 2600 is shown. Access device 2600 includes a different tissue grasping mechanism 2610, but may be used with the same stylet 2100 as described previously including tip 2604. Tissue grasping portion 2610 includes a tissue piercing tip 2604, a tissue dilating distal section 2612, a neck 2614 and a proximal stop or shoulder 2616 serving the same or similar functions as described previously. In this embodiment, however, the shoulder 2616 is movable along the length of the distal section 2612 and neck 2614, and is biased in the distal direction by biasing member (e.g., spring) 2620. hi operation, the shoulder 2616 slides back (proximal direction) as the tip 2604 and dilating distal section 2612 penetrate tissue. When the tip 2604 and dilating distal section 2612 penetrate through a fibrous tissue layer, the tissue elastically recoils around the neck 2614 and the shoulder 2616 slides forward (distal direction) to fold or plicate the fibrous tissue layer therebetween and thus provide additional retention force. When it is desired to remove the device 2600, the shoulder may be retracted back (proximal direction) to at least partially release the tissue layer therefrom.

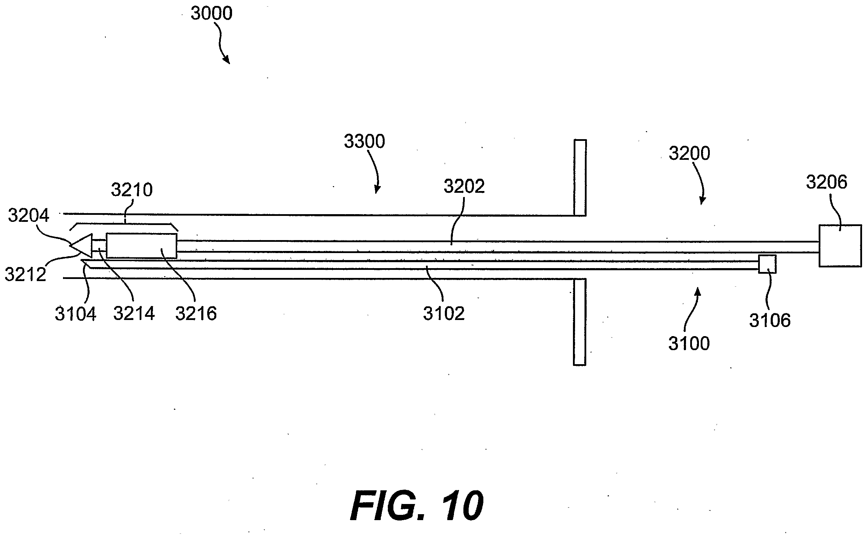

With reference to Figure 10 (not forming part of the present invention), an alternative pericardial access device 3000 is shown schematically. Access device 3000 includes a tubular stylet 3100 arranged side-by-side with a trocar member 3200. Stylet 3100 and trocar member 3200 may be disposed in a sheath 3300 to retain their proximity and to facilitate delivery through dermal and soft tissue layers leading to the access site. As compared to the access devices 1000, 2000 described previously wherein a solid stylet is disposed in a hollow trocar, access device 3000 provides a hollow stylet 3100 disposed adjacent a solid trocar 3200. Whereas the lumen in the hollow trocar 1200, 2200 of access devices 1000, 2000 provided a pathway for the guide wire, the lumen in the hollow stylet 3100 of access device 3000 provides a pathway for the guide wire to be inserted from a remote site outside the patient's body and into the pericardial space.

The hollow stylet 3100 may include an elongate shaft 3102, a distal tissue piercing tip 3104, and a proximally disposed handle 3106. The shaft 3102 and tip 3104 may comprise a metallic or rigid polymeric construction such as a stainless steel hypotube. The trocar 3200 may include an elongate shaft 3202 comprising a metallic or rigid polymeric construction and a proximally disposed handle 3206. The trocar 3200 also includes a tissue grasping portion 3210 having a tissue piercing tip 3204, a tissue dilating distal section 3212, a tissue retaining neck 3214, and a stop or shoulder portion 3216, each of which serve the same or similar function as described previously. However, in use, after the tissue grasping portion 3210 has penetrated and retained a fibrous tissue layer therein, the hollow stylet 3100 is advanced in a distal direction to pierce the tissue layer adjacent the tissue grasping portion 3210 to gain access to the pericardial space and provide a pathway thereto.

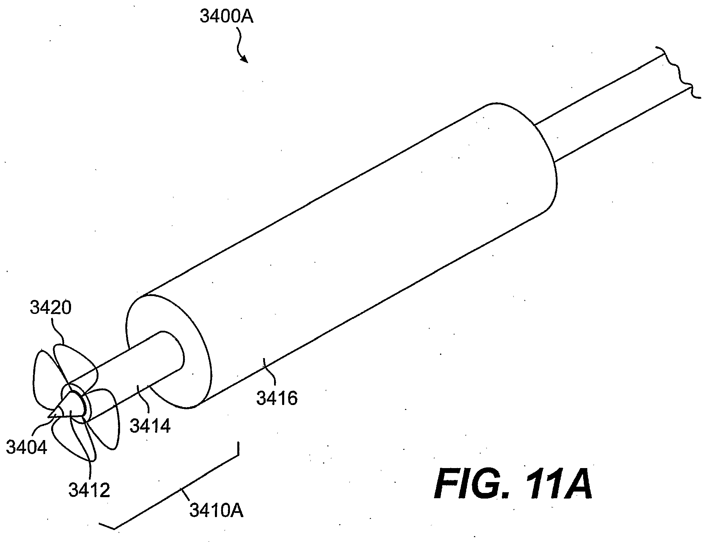

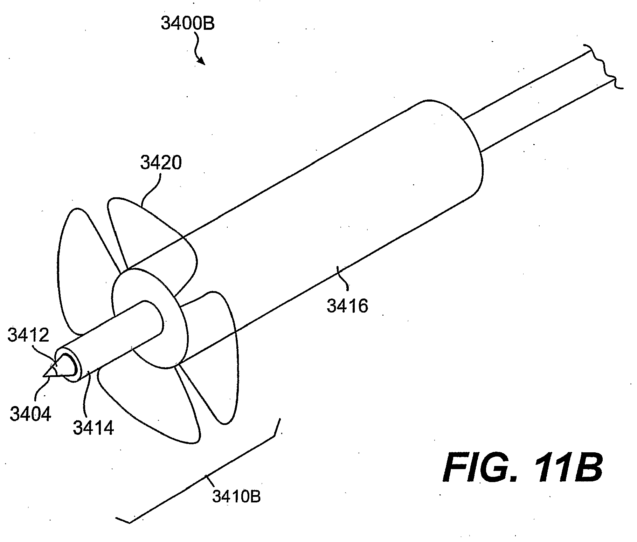

With reference to Figures 11A and 11B (not forming part of the present invention), two versions (A & B) of an alternative pericardial access device 3400 are shown. Each access device 3400A, 3400B includes a different tissue grasping mechanism 3410A, 3410B but may be used with the same stylet 2100 as described previously including tip 3404. The tissue grasping portions 3410A and 3410B each include a tissue piercing tip 3404, a tissue dilating distal section 3412, a neck 3414 and a proximal stop or shoulder 3416 serving the same or similar functions as described previously. In the embodiment shown in Figure 11A, one or more (e.g., four as shown) wings 3420 extend radially from the distal end of the neck 3414 to assist in retaining the pericardium. The wings 3420 may comprise a highly elastic or super elastic construction such as NiTi wire. In use, as the tissue grasping portion 3410A is advanced through the pericardium, the wings 3420 are elastically folded back onto the neck 3414 in their delivery configuration. When the distal end of the neck 3414 extends through the pericardium, the wings 3420 elastically expand to their deployed configuration (as shown) in the pericardial space to resist withdrawal therefrom. In the embodiment shown in Figure 11B, the wings 3420 are positioned at the distal end of the shoulder 3416 such that they expand between the pericardium and fatty deposits disposed thereon, or within such fatty deposits to assist in retaining the pericardium. In both embodiments, the wings 3420 provide an increase in surface area upon deployment to more securely retain the pericardium and/or its adjacent layers. It is contemplated that the wings 3420 may be disposed in either or both positions (e.g., distal of neck 3414 or distal of shoulder 3416) as shown in Figures 11A and 11B.

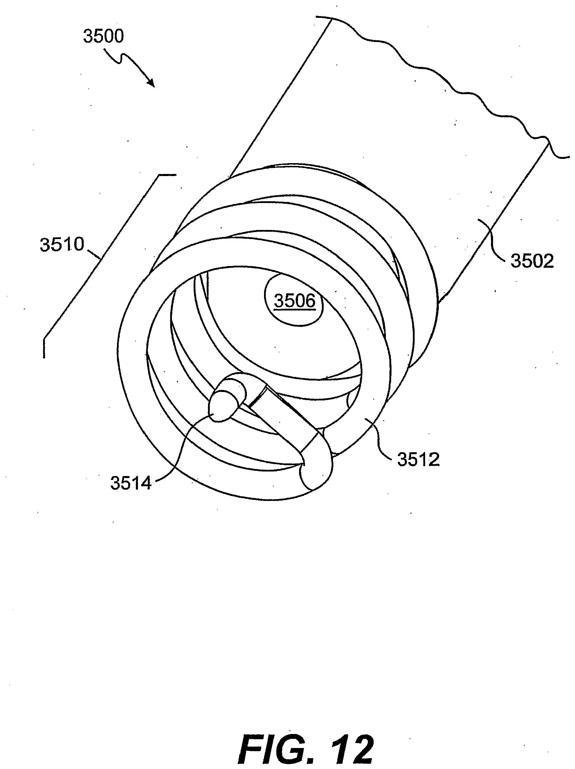

With reference to Figure 12 (not forming part of the present invention), an alternative pericardial access device 3500 is shown. Access device 3500 includes a different tissue grasping mechanism 3510, but may be used with the either stylet 2100 or hollow stylet 3100 (not shown) as described previously, which is insertable into lumen 3506 of the trocar shaft 3502. Tissue grasping portion 3510 includes a tissue piercing tip 3514 and a helical anchor 3512. In use, as the device 3500 is advanced toward the pericardium, the tip 3514 pierces the pericardium and fatty deposits disposed thereon, and the helical anchor 3512 may be screwed into the same by rotation of the shaft 3502. With the tissue grasping portion 3510 attached to the pericardium, the stylet 2100 or 3100 (not shown) may be advanced through lumen 3506, through the pericardium and into the pericardial space thus providing access thereto.

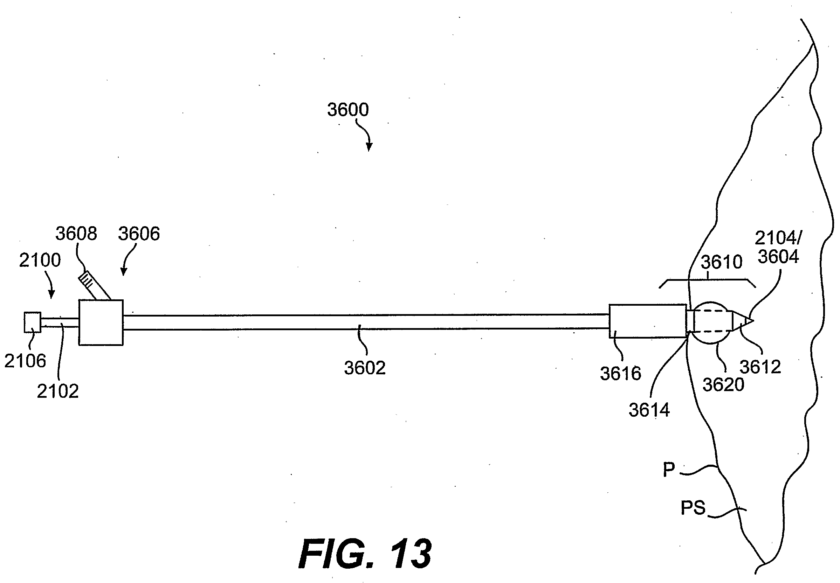

With reference to Figure 13, an alternative pericardial access device 3600 is shown. Access device 3600 includes a different tissue grasping mechanism 3610, but may be used with the same stylet 2100 as described previously, including shaft 2102, tip 2104, and hub or handle 2106. Tissue grasping portion 3610 includes a tissue piercing tip 3604/2104, a tissue dilating distal section 3612, a neck 3614 and a proximal stop or shoulder 3616 serving the same or similar functions as described previously. In this embodiment, however, the tissue grasping portion 3610 also includes an inflatable balloon 3620 disposed about a distal portion of the neck 3614. An inflation port 3608 is provided in the hub or manifold 3606 to facilitate inflation and deflation of the balloon 3620 via an inflation lumen extending through the trocar shaft 3602. In use, as the tissue grasping portion 3610 is advanced through the pericardium with the balloon 3620 in its deflated delivery configuration. When the neck 3614 extends through the pericardium, the balloon 3620 may be inflated (i.e., expanded) to its deployed configuration (as shown) in the pericardial space to resist withdrawal therefrom. After a guide wire is inserted into the pericardial space PS in place of the stylet 2100, the balloon 3620 may be deflated to facilitate removal of the device 3600.

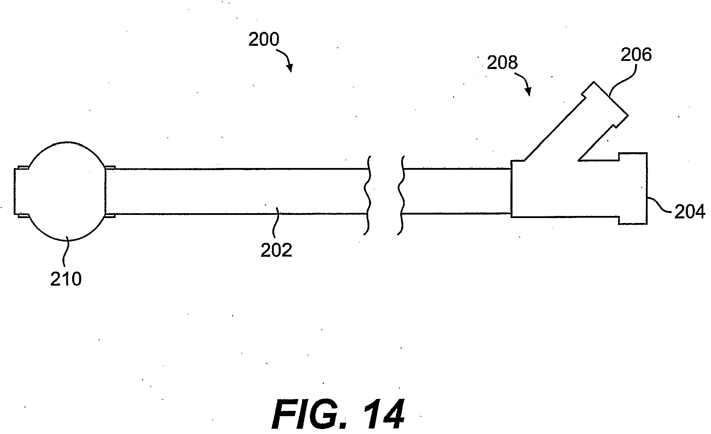

With reference to Figure 14, an access supplementation device 200 (not forming part of the present invention) is shown in the form of a sheath with a distally disposed balloon 210. The device is similar to a conventional sheath and may be used, for example, in the sub-step of access supplementation 180 as described previously. Device 200 includes a multi-lumen tubular shaft 202 with a distally disposed balloon 210 and a proximally disposed manifold 208. Manifold 208 includes a thru port 204 for insertion of devices therethrough, and an inflation port 206 for selective inflation and deflation of balloon 210. Device 200 has the particular advantage of balloon 210 which may reside is the pericardial space and resist pull-out as other devices (e.g., guide wires, dilators, therapeutic devices, etc.) are inserted therethrough.

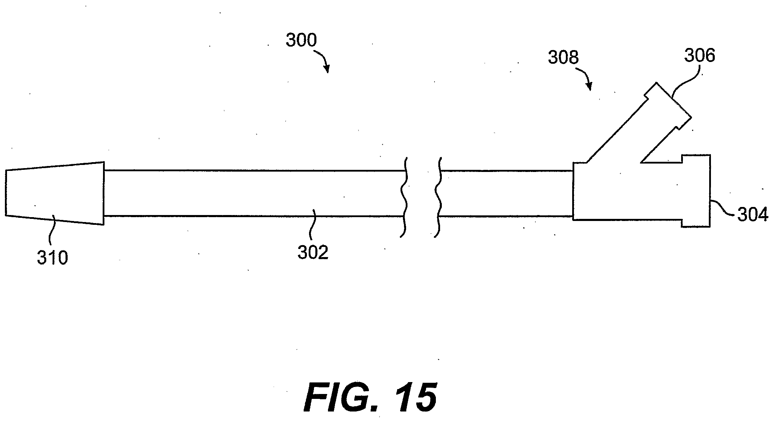

With reference to Figure 15, another access supplementation device 300 (not forming part of the present invention) is shown in the form of a dilator with a distally disposed collar 310. The device is similar to a conventional dilator and may be used, for example, in the sub-step of access supplementation 180 as described previously. Device 300 includes a tubular shaft 302 having a lumen extending therethrough, with a distally disposed collar 310 and a proximally disposed manifold 308. Manifold 308 includes a thru port 304 for insertion of devices therethrough, and an infusion port 306 for infusing fluids or flushing the shaft 302. The collar 310 has a proximal ridge that catches on the inside surface of the pericardium when the collar 310 is disposed in the pericardial space to resist pull-out as other devices (e.g., guide wires, dilators, therapeutic devices, etc.) are inserted therethrough or thereover.

Suction may also be applied to device 300 through its internal lumen to cause evacuation of the pericardial space and/or to cause the pericardium to be urged toward the heart wall. Urging the pericardium toward the heart wall aids in placing devices in intimate contact with the epicardial surface of the heart wall. Optionally, the device 300 may incorporate a steering mechanism known in the art to cause deflection of the tip and facilitate navigation in the pericardial space.

From the foregoing, it will be apparent to those skilled in the art that the present disclosure provides, in exemplary non-limiting embodiments, devices and methods for establishing pericardial access to facilitate therapeutic and/or diagnostic applications. Further, those skilled in the art will recognize that the present invention may be manifested in a variety of forms other than the specific embodiments described and contemplated herein. Accordingly, departures in form and detail may be made without departing from the scope of the present invention as described in the appended claims.

## Claims

1. An apparatus (1000) for accessing the pericardial space of the heart to perform a medical procedure, the apparatus comprising:
a shaft (1202A; 1202B) having a lumen and a distal portion, the distal portion comprising a penetration tip (1104) and a tissue grasping portion (1210) disposed proximal the penetration tip (1104), the tissue grasping portion (1210) comprising a dilating member (1212) proximal the penetration tip (1104) so as to provide a smooth transition between the dilating member (1212) and the penetration tip (1104), a region of reduced cross-section (1214) proximal and fixedly attached to the dilating member (1212), and a region of enlarged cross-section (1216) proximal the region of reduced cross-section (1214) and configured to abut the pericardium such that it cannot be inserted past the pericardium, wherein the lumen extends through the tissue grasping portion (1210) so as to provide access to the pericardial space from a location remote from the pericardial space,
a stylet (1100) configured to be inserted through the lumen of the shaft (1202A; 1202B), wherein the penetration tip (1104) is on the stylet (1100),
**characterized in that** the region of enlarged cross-section (1216) is fixedly attached to the region of reduced cross-section, and a profile (C) of the penetration tip (1104), a profile (A, C, E) of the dilation member (1212), a profile (B, D) of the region of reduced cross-section (1214), and a profile (F) of the region of enlarged cross-section (1216) are selected such that the distal portion is configured to be automatically inserted through the pericardium to a predetermined depth beyond the pericardium and to separate the pericardium from the epicardium.

2. The apparatus of claim 1, wherein the region of enlarged cross-section (1216) includes a shoulder.

3. The apparatus of claim 1, wherein the profile (A, C, E) of the dilating member (1212) is further selected such that once the dilation member (1212) is inserted past the pericardium, moving the distal portion in a proximal direction causes the dilation member (1212) to separate the pericardium from the epicardium.

4. The apparatus of claim 1, wherein the profile (A, C, E) of the dilating member (1212) is further selected such that the dilation member (1212) is configured to elastically dilate the pericardium.

5. The apparatus of claim 4, wherein the profile (A, C, E) of the dilating member (1212) is further selected such that the dilation member (1212) is configured to elastically dilate the pericardium by an amount such that the dilation member (1212) can separate the pericardium from the epicardium without tearing the pericardium upon moving the distal portion in a proximal direction.

6. The apparatus of claim 1, wherein the profile (A, C, E) of the dilating member (1212) is further selected such that the dilation member (1212) is configured to dilate the pericardium during insertion by an amount such that the pericardium elastically recoils around the region of reduced cross-section (1214).

7. The apparatus of claim 1, wherein the penetration tip (1104) is configured to pierce the pericardium.

## Patentansprüche

1. Vorrichtung (1000) zum Zugreifen auf den perikardialen Raum des Herzens zur Durchführung eines medizinischen Eingriffs, wobei die Vorrichtung umfasst:
einen Schaft (1202A; 1202B) mit einem Lumen und einem distalen Abschnitt, wobei der distale Abschnitt eine Penetrierspitze (1104) und einen distal von der Penetrierspitze (1104) angeordneten, gewebefassenden Abschnitt (1210) umfasst, wobei der gewebefassende Abschnitt (1210) umfasst: ein proximal von der Penetrierspitze (1104) befindliches Erweiterungselement (1212), um einen glatten Übergang zwischen dem Erweiterungselement (1212) und der Penetrierspitze (1104) bereitzustellen, einen proximal von dem Erweiterungselement (1212) befindlichen und an diesem fest angebrachten Bereich (1214) mit verringertem Querschnitt, und einen proximal von dem Bereich (1214) mit verringertem Querschnitt befindlichen Bereich (1216) mit vergrößertem Querschnitt, der dafür ausgelegt ist, an dem Perikard anzustoßen, so dass er nicht weiter als bis an das Perikard eingesetzt werden kann, wobei sich das Lumen durch den gewebefassenden Abschnitt (1210) hindurch erstreckt, um dadurch von einem von dem perikardialen Raum entfernten Ort aus einen Zugang zu dem perikardialen Raum bereitzustellen,
ein Stilett (1100), das dafür ausgelegt ist, durch das Lumen in dem Schaft (1202A; 1202B) hindurchgeführt zu werden, wobei sich die Penetrierspitze (1104) auf dem Stilett (1100) befindet,
**dadurch gekennzeichnet, dass** der Bereich (1216) mit vergrößertem Querschnitt fest an dem Bereich mit verringertem Querschnitt angebracht ist und ein Profil (C) der Penetrierspitze (1104), ein Profil (A, C, E) des Erweiterungselements (1212), ein Profil (B, D) des Bereichs (1214) mit verringertem Querschnitt, und ein Profil (F) des Bereichs (1216) mit vergrößertem Querschnitt derart ausgewählt sind, dass der distale Abschnitt dafür ausgelegt ist, dass er automatisch durch das Perikard hindurch bis zu einer vorbestimmten Tiefe über das Perikard hinausgehend eingesetzt wird und das Perikard vom Epikard trennt.

2. Vorrichtung nach Anspruch 1, wobei der Bereich (1216) mit vergrößertem Querschnitt eine Schulter umfasst.

3. Vorrichtung nach Anspruch 1, wobei das Profil (A, C, E) des Erweiterungselements (1212) ferner derart ausgewählt ist, dass sobald das Erweiterungselement (1212) über das Perikard hinausgehend eingesetzt ist, ein Bewegen des distalen Abschnitts in einer proximalen Richtung bewirkt, dass das Erweiterungselement (1212) das Perikard vom Epikard trennt.

4. Vorrichtung nach Anspruch 1, wobei das Profil (A, C, E) des Erweiterungselements (1212) ferner derart ausgewählt ist, dass das Erweiterungselement (1212) dafür ausgelegt ist, das Perikard elastisch zu erweitern.

5. Vorrichtung nach Anspruch 4, wobei das Profil (A, C, E) des Erweiterungselements (1212) ferner derart ausgewählt ist, dass das Erweiterungselement (1212) dafür ausgelegt ist, das Perikard in einem solchen Ausmaß elastisch zu erweitern, dass das Erweiterungselement (1212) das Perikard vom Epikard trennen kann, ohne dabei das Perikard beim Bewegen des distalen Abschnitts in einer proximalen Richtung zu zerreißen.

6. Vorrichtung nach Anspruch 1, wobei das Profil (A, C, E) des Erweiterungselements (1212) ferner derart ausgewählt ist, dass das Erweiterungselement (1212) dafür ausgelegt ist, das Perikard während des Einsetzens in einem solchen Ausmaß zu erweitern, dass das Perikard elastisch um den Bereich (1214) mit verringertem Querschnitt herum zurückschnellt.

7. Vorrichtung nach Anspruch 1, wobei die Penetrierspitze (1104) dafür ausgelegt ist, das Perikard zu durchstechen.

## Revendications

1. Dispositif (1000) permettant l'accès à l'espace péricardique du coeur en vue de réaliser une intervention médicale, lequel dispositif comprend :
un corps (1202A ; 1202B) avec une lumière et une partie distale, la partie distale comprenant une pointe de pénétration (1104) et une partie de préhension de tissu (1210) disposée du côté distal par rapport à la pointe de pénétration (1104), la partie de préhension de tissu (1210) comprenant : un élément de dilatation (1212) situé du côté proximal par rapport à la pointe de pénétration (1104) afin de fournir une transition douce entre l'élément de dilatation (1212) et la pointe de pénétration (1104), une zone (1214) de section transversale réduite située du côté proximal par rapport à l'élément de dilatation (1212) et montée de manière fixe sur ce dernier, ainsi qu'une zone (1216) de section transversale agrandie située du côté proximal par rapport à la zone (1214) de section transversale réduite et conçue pour heurter le péricarde, de sorte qu'elle ne peut pas être introduite au-delà dudit péricarde, la lumière s'étendant à travers la partie de préhension de tissu (1210) afin de fournir un accès à l'espace péricardique depuis un point éloigné de l'espace péricardique,
un stylet (1100) qui est conçu pour être inséré à travers la lumière prévue dans le corps (1202A ; 1202B), la pointe de pénétration (1104) étant située sur le stylet (1100),
**caractérisé en ce que** la zone (1216) de section transversale agrandie est montée de manière fixe sur la zone de section transversale réduite et un profil (C) de la pointe de pénétration (1104), un profil (A, C, E) de l'élément de dilatation (1212), un profil (B, D)de la zone (1214) de section transversale réduite et un profil (F) de la zone (1216) de section transversale agrandie sont choisis de sorte que la partie distale est conçue pour être automatiquement introduite à travers le péricarde et aller au-delà de celui-ci jusqu'à une profondeur prédéterminée et pour séparer le péricarde de l'épicarde.

2. Dispositif selon la revendication 1, dans lequel la zone (1216) de section transversale agrandie comprend un épaulement.

3. Dispositif selon la revendication 1, dans lequel le profil (A, C, E) de l'élément de dilatation (1212) est en outre choisi de sorte que, une fois que l'élément de dilatation (1212) a dépassé le péricarde, un déplacement de la partie distale dans une direction proximale fait en sorte que l'élément de dilatation (1212) sépare le péricarde de l'épicarde.

4. Dispositif selon la revendication 1, dans lequel le profil (A, C, E) de l'élément de dilatation (1212) est en outre choisi de sorte que l'élément de dilatation (1212) est conçu pour dilater le péricarde de manière élastique.

5. Dispositif selon la revendication 4, dans lequel le profil (A, C, E) de l'élément de dilatation (1212) est en outre choisi de sorte que l'élément de dilatation (1212) est conçu pour dilater le péricarde de manière élastique de telle sorte que ledit élément de dilatation (1212) puisse séparer le péricarde de l'épicarde sans pour autant déchirer le péricarde lorsque la partie distale est déplacée dans une direction proximale.

6. Dispositif selon la revendication 1, dans lequel le profil (A, C, E) de l'élément de dilatation (1212) est en outre choisi de sorte que ledit élément de dilatation (1212) est conçu pour dilater le péricarde de manière telle que, lorsqu'il est introduit, le péricarde recule en s'enroulant élastiquement autour de la zone (1214) de section transversale réduite.

7. Dispositif selon la revendication 1, dans lequel la pointe de pénétration (1104) est conçue pour transpercer le péricarde.
